# EUROPEAN PATENT SPECIFICATION

(11) **EP 0 382 268 B1**
(45) Date of publication and mention of the grant of the patent: **24.08.1994**
(21) Application number: 90200031.4
(22) Date of filing: 04.01.1990
(51) Int. Cl.: C07D 501/18, C07D 499/02, C07C 251/02

(54) **New amino carboxylic acid derivatives**
Derivate von Aminocarboxylsäure
Dérivés d'acides aminocarboxyliques

(30) Priority: 06.01.1989 EP 89200034
(43) Date of publication of application: 16.08.1990
(73) Proprietor: GIST-BROCADES N.V., NL-2600 MA Delft (NL)
(72) Inventor: Verweij, Jan, NL-2313 ES Leiden (NL); Hirs, Henri Gerard Julius, NL-2717 DD Zoetermeer (NL); Witkamp, Hendrik Adrianus, NL-2641 VG Pijnacker (NL)
(74) Representative: Visser-Luirink, Gesina, Dr.

(56) References cited:
- EP-A- 0 300 546
- DE-A- 2 440 142
- US-A- 3 821 198

## Description

The invention relates to new amino acid derivatives and to processes for the preparation of the same.

In European patent application 300546, filed on July 8, 1988, a process for the preparation of new 7β-(cyclo)alkylideneammonio-4-carboxy-3-cephem-3-halomethyl halide compounds is described. These new compounds are formed during a one pot process for the preparation of 7-amino-3-cephem-3-substituted methyl-4-carboxylic acid derivatives from 7-acylamino-3-cephem-3-methyl-4-carboxylic acid derivatives by adding a ketone and, if necessary, an acid, to the reaction mixture.

In the literature, the formation of (cyclo)alkylideneammonio carboxylic acid derivatives as defined further herein has not previously been described. Methylene ammonio cephem compounds and the preparation of methyl amine compounds by reduction of the same have been disclosed in U.S. patent 3,821,198. In the German patent application 2440142 halomethylene ammonio cephem compounds have been mentioned, suitable to introduce thiogroups on the 7-position of the cephem compound. Apart from the new cephem derivatives mentioned in European patent application 300546 the only other cephalosporanic acid derivatives referred to in the literature are those having an aldimine substituent at the 7-position as described, for instance, by W.A. Spitzer, T. Goodson, R.J. Smithey and J.G. Wright, J. Chem. Soc. Chem. Comm., 1338 (1972).

It has now surprisingly been found that in general the N-terminal amino group of an amino carboxylic acid, which is stable in acid medium, can react with a ketone in acid medium to form a (cyclo)alkylideneammonio group.

Therefore, in accordance with the present invention, there is provided a new compound which is an amino carboxylic acid derivative comprising, as an N-terminal group, a (cyclo)alkylideneammonio group of formula (II): 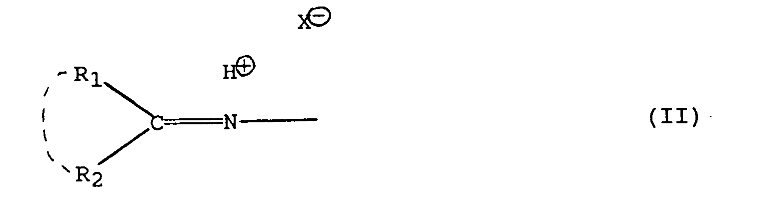
wherein
X^{⊖} is an anion from an acid HX,
R₁ and R₂ are the same or different and each is a C₁-C₁₆ alkyl group or R₁ and R₂, together with the carbon atom to which they are attached, form a cycloalkylidene ring with up to 8 carbon atoms, with the proviso that the compound is not 7β-(cyclo)alkylideneammonio-3-halomethyl-3-cephem-4-carboxylic acid.

In particular, compounds of the invention include β-lactam derivatives and more in particular those of formula (I): 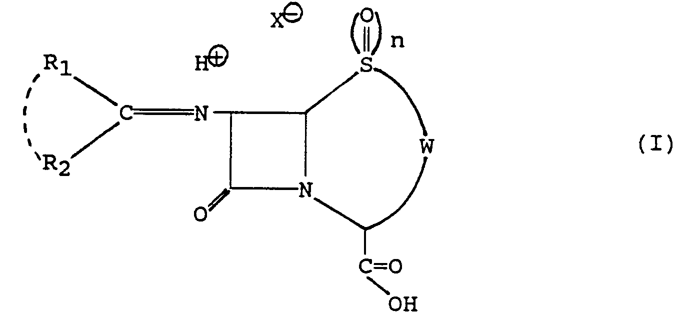
wherein
W is 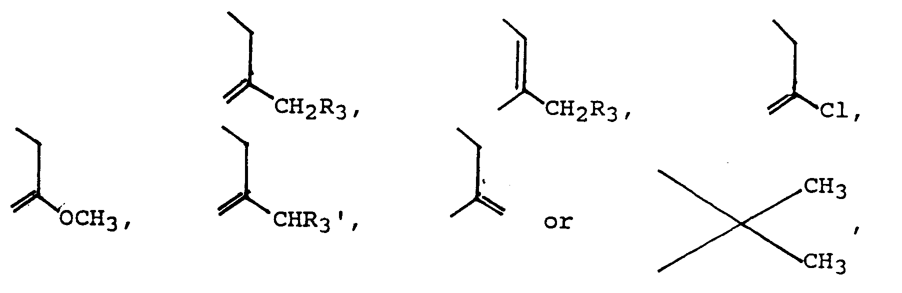
R₃ is hydrogen, (lower)alkoxy, (lower)alkylthio, (lower)alkanoyloxy, (lower)alkanoylthio or S-R₄, where R₄ is an optionally substituted heterocyclic ring,
R₃' is (lower)alkylidene,
n is 0, 1 or 2 and
R₁, R₂ and X are as defined above.

Preferred compounds of formula (I) include those wherein
W is 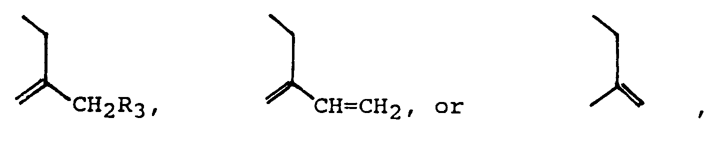
where R₃ is hydrogen, acetoxy, (1-methyl-1H-tetrazol-5-yl)thio and n is 0 or 1 or
W is 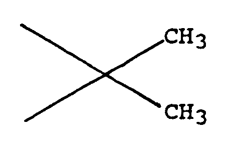
and n is 2,
X is Cl⁻, Br⁻, I⁻, ClO₄⁻, HSO₄⁻, CH₃COO⁻, and
R₁ is methyl, R₂ is methyl or R₁ and R₂ together with the carbon atom to which they are attached form cyclopentylidene or cyclohexylidene.

Furthermore, a process is provided for the preparation of an amino carboxylic acid derivative wherein the N-terminal amine group of an amino carboxylic acid is converted into a group of formula (II) as defined above by adding a ketone R₁R₂CO to the amino carboxylic acid in the presence of an acid HX, R₁, R₂ and X being as defined for formula (II) above. Any amino carboxylic acid which is stable in acid medium can be used as starting material.

In one embodiment of the invention a new compound which is an amino carboxylic acid derivative as defined above in accordance with the invention is prepared by deprotecting an N-protected amino carboxylic acid in a manner known per se and then by adding a ketone R₁R₂ in situ. The N-protected amino carboxylic acid is an amino carboxylic acid bearing any conventional protected amino group at its N-terminus.

In particular, a process is provided for the preparation of an amino acid derivative of formula (I), as defined above, from an amino carboxylic acid of the following formula (III): 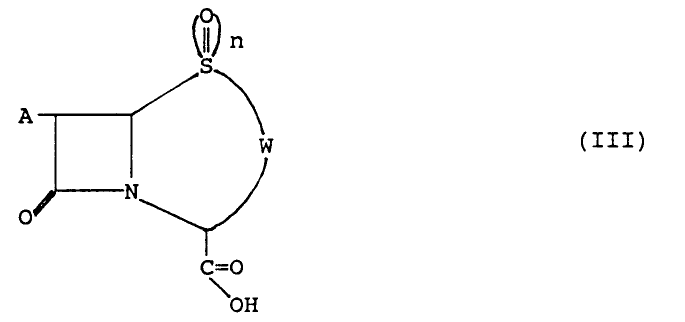
wherein
W and n are as defined above in general formula (I), and A is a protected amino group,
by carrying out the following successive steps:
(a) silylating the starting amino carboxylic acid, optionally in situ,
(b) converting the product of step (a) into an imidoyl chloride,
(c) reacting the imidoyl chloride with an alcohol to form the corresponding iminoether and/or the 7-amino derivative, and
(d) adding a ketone R₁R₂CO.

The silylation of step (a) is achieved by any suitable known procedure to split off side chains in cephem and penem compounds, for example by treating the starting amino carboxylic acid with trimethylchloro silane or dimethyldichloro silane, and a base like for instance N,N-dimethylaniline, pyridine or quinoline, in methylene chloride. Step (b) is also carried out by any suitable known method such as treatment with phosphorus pentachloride at a low temperature, for example -70°C to +5°C. In step (c) the imidoyl chloride may be reacted with any alcohol, suitable examples of which include methanol, ethanol, isobutanol and 1,3-propanediol.

In one embodiment all four steps are performed in the same reaction vessel without isolation of the intermediate products. It is alternatively possible to separate the product of one step before performing the next step. All four reaction steps are carried out at a temperature in the range from -80°C to +150°C, preferably from -60°C to +100°C.

As used herein, the terms lower alkyl and (lower)alkyl refer to an alkyl group with up to 8 carbon atoms. By Rₙ₋ₙ₊ₘ is meant: Rₙ, Rₙ₊₁.....Rₙ₊ₘ. The (cyclo)alkylideneammonio group can alternatively be referred to as a (cyclo)alkylideneiminium group.

The term amino carboxylic acid as used herein refers to a compound comprising at least a primary amino group and a carboxylic acid group. If the primary amino group has been N-protected, the protective group can be hydrolysed in conventional fashion. Conversion to an N-(cyclo)alkylideneammonio group can then be effected by adding a ketone.

If necessary, an acid medium can be created by the addition of any inorganic or organic acid. The acid is typically added as a concentrated solution. Suitable acids include hydrogen chloride, hydrogen bromide, hydrogen iodide, sulphuric acid, perchloric acid and acetic acid.

When R₃ is the group S-R₄, the heterocyclic ring R₄ is suitably pyridine, pyrimidine, pyridazine, pyrrole, imidazole, pyrazole, isoxazole, isothiazole, thiazole, triazole, oxadiazole, thiadiazole, triazine, thiatriazole or tetrazole linked by a ring carbon atom to the sulphur atom. The heterocyclic ring R₄ is unsubstituted or substituted on a ring carbon atom, examples of suitable substituents including optionally substituted lower alkyl, cyano, chloro, di(lower)alkylamino, (lower)alkyloxy such as methoxy, (lower)alkyloxycarbonyl, di(lower)alkylcarbamoyl, hydroxy, sulfo and carboxy. One or more ring nitrogen atoms of the heterocyclic group R₄ can be substituted by an optionally substituted lower alkyl group. Examples of suitable substituents attached to the first or the second carbon atom of a lower alkyl group attached either to a ring carbon or ring nitrogen atom of the heterocyclic ring R₄ include di(lower)alkylamino, chloro, cyano, methoxy, (lower)alkyloxycarbonyl, N,N-dimethylcarbamoyl, hydroxy, carboxy and sulfo.

Conventional "protected amino groups" as used in this specification include amino groups of the formula 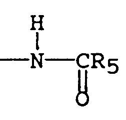
which can by introduced by cephalosporin and penicillin fermentation, R₅ being an optionally substituted methyl group -CH₂R₆ which can be introduced by penicillin fermentation and wherein R₆ is hydrogen, aryl, alkyl, cycloalkyl, alkenyl, aryloxy, arylthio, alkylthio, etc., or R₅ is an optionally substituted aryl group.

The present invention provides a new and general method of protecting an amino group as a (cyclo)alkylideneammonio group, which is a powerful means in synthesis wherein amino carboxylic acids have to be protected. Compounds of the invention are therefore useful as synthetic intermediates.

Among these compounds β-lactam derivatives are valuable as intermediates in the preparation of various therapeutically useful antibiotics. For instance, 3-acetoxymethyl-4-carboxy-7-(cyclo)alkylideneammonio-3-cephem bromide compounds of the invention may be converted into 7-amino-3-substituted methyl-3-cephem-4-carboxylic acid derivatives such as 7-amino-3-[[1-methyl-1H-tetrazol-5-yl)thio]methyl]-3-cephem-4-carboxylic acid and 7-ammonio-4-carboxy-3-[1-pyridinio)methyl]-3-cephem diiodide.

The following Examples further illustrate the invention.

Examples I-IX show the preparation of certain new (cyclo)alkylideneammonio-3-acetoxymethyl-3-cephem compounds and Examples X-XV the preparation of certain new (cyclo)alkylideneammonio-3-methyl-3-cephem compounds.
Examples XVI-XVIII illustrate the preparation of new (cyclo)alkylideneammonio-3-[[(1-methyl-1H-tetrazol-5-yl)thio]methyl]-3-cephem derivatives and Examples XIX-XXI the preparation of new (cyclo)alkylideneammonio-3-vinyl-3-cephem derivatives.
Example XXII shows the preparation of a (cyclo)alkylidene-1-oxo-3-cephem, Example XXIII of a (cyclo)alkylideneammonio-1,1-dioxo-penam, Example XXIV of a (cyclo)alkylideneammonio-2-cephem, Example XXV of a (cyclo)alkylidene-3-methylenecepham and Example XXVI of a (cyclo)alkylidene-β-alaninium derivative.
The Examples XXVII-XXX show "one-pot" syntheses of (cyclo)alkylideneammonio-3-cephem compounds from corresponding protected amino-3-cephem compounds.
Finally, Examples XXXI and XXXII illustrate the conversion of these new (cyclo)alkylideneammonio compounds into the corresponding amino derivatives.

### Example I

### Preparation of 3-acetoxymethyl-4-carboxy-7-isopropylideneammonio-3-cephem chloride from 7-amino-3-acetoxymethyl-3-cephem-4-carboxylic acid

After addition of 20.0 g (purity 90%; 70.5 mmoles) of 7-amino-3-acetoxymethyl-3-cephem-4-carboxylic acid to 50 ml of acetic acid saturated with hydrochloric acid at 20°C a clear solution was obtained in about 3 minutes. Then 110 ml of acetone were added, the mixture was cooled down and a white precipitate was formed.
After stirring for another 30 minutes and adding 80 ml of acetone the precipitate was filtered off, washed with acetone and dried giving 26.5 g (purity 90%) of the title product. Yield 97%.

IR (KBr-disc, values in cm⁻¹): 3000, 2820, 2570, 1980, 1785, 1720, 1665, 1635, 1500, 1380, 1340, 1225, 1160, 1115, 1035, 975, 920, 875, 810, 720, 700, 440.

NMR (360 MHz; CF₃COOD; tetramethylsilane as a reference; δ-values in ppm): 1.99 (s, 3H); 2.52 (s, 3H); 2.62 (s, 3H); 3.45, 3.55 (AB-q, 2H; J = 18 Hz); 4.98, 5.20 (AB-q, 2H; J = 14.4 Hz); 5.27 (d, 1H; J = 4.6 Hz); 5.84 (d, 1H; J = 4.6 Hz).

### Example II

### Preparation of 3-acetoxymethyl-4-carboxy-7-isopropylideneammonio-3-cephem bromide from 7-amino-3-acetoxymethyl-3-cephem-4-arboxylic acid

To a mixture of 1.0 g (purity 97%; 3.56 mmoles) of 7-amino-3-acetoxymethyl-3-cephem-4-carboxylic acid and 0.8 ml of a HBr solution in acetic acid (33%), 5 ml of acetone were added gradually. The precipitate formed was filtered off, washed with acetone and dried, giving 0.85 g of the title product.

IR (KBr-disc, values in cm⁻¹): 3185, 3050, 2940, 2905, 2715, 1795, 1735, 1710, 1665, 1625, 1520, 1415, 1405, 1375, 1335, 1230, 1215, 1195, 1165, 1110, 1060, 1040, 975, 920, 820, 790, 720, 700, 440.

NMR (360 MHz; CF₃COOD; tetramethylsilane as a reference; δ-values in ppm): 1.99 (s, 3H); 2.53 (s, 3H); 2.63 (s, 3H); 3.49, 3.56 (AB-q, 2H; J = 18 Hz); 5.00, 5.19 (AB-q, 2H; J = 14.4 Hz); 5.33 (d, 1H; J = 4.6 Hz); 5.88 (d, 1H; J = 4.6 Hz).

### Example III

### Preparation of 3-acetoxymethyl-4-carboxy-7-isopropylideneammonio-3-cephem bromide from 7-amino-3-acetoxymethyl-3-cephem-4-carboxylic acid

To a suspension of 10.0 g (purity 96%; 35.3 mmoles) of 7-amino-3-acetoxymethyl-3-cephem-4-carboxylic acid in 100 ml of acetone and 20 ml of acetic acid, 6.7 ml of a HBr solution in water (47%) were added. After stirring for 210 minutes at 0°C the precipitate formed was filtered off, washed with acetone and dried in vacuo, giving 10.77 g (purity 100%) of the title product. Yield 77%.

### Example IV

### Preparation of 3-acetoxymethyl-4-carboxy-7-isopropylideneammonio-3-cephem bromide from 7-amino-3-acetoxymethyl-3-cephem-4-carboxylic acid

To a suspension of 10.0 g (purity 96%; 35.5 mmoles) of 7-amino-3-acetoxymethyl-3-cephem-4-carboxylic acid in 50 ml of acetone, 9.8 ml of a HBr solution in acetic acid (33%) were added at 0°C during 5 minutes. After stirring for 60 minutes at 0°C the precipitate formed was filtered off, washed with acetone (3 x 25 ml) and dried in vacuo, giving 13.7 g (purity 93%) of the title product. Yield 91%.

### Example V

### Preparation of 3-acetoxymethyl-4-carboxy-7-isopropylideneammonio-3-cephem iodide from 7-amino-3-acetoxymethyl-3-cephem-4-carboxylic acid

To a suspension of 5 g (17.9 mmoles) of 7-amino-3-acetoxymethyl-3-cephem-4-carboxylic acid in a mixture of 10 ml of acetone and 5 ml of acetic acid, 4.0 ml of a hydrogen iodide solution in water (57%) were added. After stirring for about 90 minutes at 0°C, the crystals formed were filtered off, washed with acetone and dried giving 2.69 g of the title product.

IR (KBr-disc, values in cm⁻¹): 3180, 3040, 2900, 2760, 1795, 1740, 1715, 1675, 1630, 1510, 1415, 1400, 1380, 1230, 1205, 1165,1115, 1065, 1045, 975, 920, 840, 815, 785, 715, 700, 440.

NMR (360 MHz; CF₃COOD; tetramethylsilane as a reference; δ-values in ppm): 1.99 (s, 3H); 2.53 (s, 3H); 2.64 (s, 3H); 3.50, 3.57 (AB-q, 2H; J = 18 Hz); 5.00, 5.19 (AB-q, 2H; J = 14.4 Hz); 5.35 (d, 1H; J = 4.6 Hz); 5.89 (d, 1H; J = 4.6 Hz).

### Example VI

### Preparation of 3-acetoxymethyl-4-carboxy-7-cyclopentylideneammonio-3-cephem bromide from 7-amino-3-acetoxymethyl-3-cephem-4-carboxylic acid

In a centrifuge tube 0.8 ml of a hydrogen bromide solution (33%) in acetic acid was added to 1.0 g (purity 96%; 3.53 mmoles) of 7-amino-3-acetoxymethyl-3-cephem-4-carboxylic acid at 0°C with stirring. After adding gradually 5 ml of cyclopentanone a crystalline precipitate was obtained after 30 minutes, which was filtered off, washed with acetone and cyclopentanone, and dried giving 1.17 g (80%) of the title product.

IR (KBr-disc, values in cm⁻¹): 3180, 3000, 2900, 2630, 1795, 1740, 1715, 1680, 1630, 1500, 1415, 1380, 1340, 1235, 1210, 1160, 1110, 1070, 1045, 975, 920, 825, 790, 715, 455.

NMR (360 MHz; CF₃COOD; tetramethylsilane as a reference; δ-values in ppm): 2,02 (s, 3H); 2.02 (m, 4H); 3.02 (m, 4H); 3.52 and 3.58 (AB-q, 2H; J = 18.4 Hz); 5.01, 5.23 (AB-q, 2H; J = 14.1 Hz); 5.31 (d, 1H; J = 4.3 Hz); 5.75 (d, 1H; J = 4.3 Hz).

### Example VII

### Preparation of 3-acetoxymethyl-4-carboxy-7-cyclohexylideneammonio-3-cephem chloride from 7-amino-3-acetoxymethyl-3-cephem-4-carboxylic acid

A mixture of 165 mg (0.606 mmole) of 7-amino-3-acetoxymethyl-3-cephem-4-carboxylic acid, 0.08 ml of a concentrated HCl solution and 0.10 ml (0.96 mmole) of cyclohexanone was stirred vigorously in an ultrasonic bath for 15 minutes. During the next 80 minutes more cyclohexanone (0.2 ml) was added in 2 portions. Collection and washing of the precipitate with acetonitrile and ether afforded 197 mg (84%) of the title product.

IR (KBr-disc; values in cm⁻¹): 3440, 1804, 1731, 1715, 1656, 1620, 1525, 1418, 1380, 1229, 1065, 1039, 876, 721 and 704.

NMR (360 MHz; CF₃COOD; tetramethylsilane as a reference; δ-values in ppm): 1.66 (m, 2H); 1.92 (m, 4H); 1.99 (s, 3H); 2.77 (m, 4H); 3.45 and 3.57 (2xd, 2H; J = 18.0 Hz); 4.99 and 5.19 (2xd, 2H; J = 14.4 Hz); 5.27 (d, 1H; J = 4.4 Hz); 5.87 (d, 1H; J = 4.4 Hz).

### Example VIII

### Preparation of 3-acetoxymethyl-4-carboxy-7-cyclohexylideneammonio-3-cephem bromide from 7-amino-3-acetoxymethyl-3-cephem-4-carboxylic acid

To 165 mg (purity 96%; 0.58 mmole) of 3-acetoxymethyl-7-amino-cephem-carboxylate was added 0.12 ml of a concentrated HBr solution and 0.1 ml of cyclohexanone at 0°C with vigorous stirring. When stirring was continued initially a clear reaction mixture was obtained but after some time a thick crystalline precipitate was formed. After adding 0.5 ml of cyclohexanone and stirring for another 30 minutes the crystalline precipitate was filtered off and dried giving 234 mg of the title product with a purity of 93%. Yield 92%.

IR (KBr-disc; values in cm⁻¹): 3440, 2945, 2850, 1810, 1731, 1651, 1625, 1510, 1417, 1230, 1196, 1067, 1041, 833 and 702.

NMR (360 MHz; CF₃COOD; tetramethylsilane as a reference; δ-values in ppm): 1.70 (m, 2H); 1.93 (m, 4H); 1.99 (s, 3H); 2.69 (m, 4H); 3.28 and 3.36 (2xd, 2H; J = 17 Hz); 4.57 and 4.74 (2xd, 2H; J = 12 Hz); 4.85 (d, 1H; J = 4.5 Hz); 5.36 (d, 1H; J = 4.5 Hz).

### Example IX

### Preparation of 3-acetoxymethyl-4-carboxy-7-cyclohexylideneammonio-3-cephem hydrogen sulphate from 7-amino-3-acetoxymethyl-3-cephem-4-carboxylic acid

To a solution of 1.0 g (3.53 mmoles) of 7-amino-3-acetoxy methyl-3-cephem-4-carboxylic acid in 5 ml of acetic acid and 0.2 ml of sulphuric acid were added 5 ml of cyclohexanone. After standing overnight at 2°C the crystalline precipitate formed was filtered off, washed with cyclohexanone and acetone and dried in vacuo yielding 1.41 g (89%) of the title product.

IR (KBr-disc, values in cm⁻¹): 2960, 2880, 1810, 1725, 1710, 1665, 1535, 1410, 1390, 1360, 1310, 1280, 1230, 1165, 1050, 980, 850, 710, 580, 440.

NMR (360 MHz; CF₃COOD; tetramethylsilane as a reference; δ-values in ppm): 1.68 and 1.94 (m, 6H); 2.02 (s, 3H); 2.79 (m,4H); 3.45 and 3.61 (AB-q, 2H; J = 17.7 Hz); 5.01, 5.20 (AB-q, 2H; J = 14.1 Hz); 5.31 (d, 1H; J = 4.3 Hz); 5.90 (d, 1H; J = 4.3 Hz).

### Example X

### Preparation of 4-carboxy-7-isopropylideneammonio-3-methyl-3-cephem chloride from 7-amino-3-methyl-3-cephem-4-carboxylic acid

To a mixture of 1.0 g (purity 98%; 4.57 mmoles) of 7-amino-3-methyl-3-cephem-4-carboxylic acid and 2.5 ml of acetic acid saturated with hydrogen chloride, 3 ml of acetone were gradually added with vigorous stirring. After stirring for 10 minutes another 5 ml of acetone were added, the reaction mixture was stored in the refrigerator for 2 hours and the precipitate formed was filtered off, washed with acetone and dried, giving 1.26 g of the title product with a purity of 100% according to NMR assay. Yield 95%.

IR (KBr-disc, values in cm⁻¹): 3010, 2660, 1985, 1780, 1720, 1665, 1625, 1535, 1415, 1395, 1365, 1285, 1205, 1185, 1115, 1080, 1050, 1000, 970, 955, 910, 880, 800, 740, 715, 690, 565, 510.

NMR (360 MHz; CF₃COOD; tetramethylsilane as a reference; δ-values in ppm): 2.18 (s, 3H); 2.51 (s, 3H); 2.62 (s, 3H); 3.24, 3.33 (AB-q, 2H; J= 18 Hz); 5.25 (d, 1H; J = 4.3 Hz); 5.71 (d, 1H; J = 4.3 Hz).

### Example XI

### Preparation of 4-carboxy-7-isopropylideneammonio-3-methyl-3-cephem chloride from 7-amino-3-methyl-3-cephem-4-carboxylic acid

With stirring 1.0 g (purity 98%; 4.57 mmoles) of 7-amino-3-methyl-3-cephem-4-carboxylic acid was added to a mixture of 10 ml of acetic acid, 10 ml of acetone and 0.45 ml of a HCl solution in water (37%) at 0°C. Stirring for 45 minutes, filtration of the precipitate formed, washing with acetone and drying yielded 1.16 g (87%) of the title product.

### Example XII

### Preparation of 4-carboxy-7-isopropylideneammonio-3-methyl-3-cephem bromide from 7-amino-3-methyl-3-cephem-4-carboxylic acid

To a mixture of 1.0 g (purity 98%; 4.57 mmoles) of 7-amino-3-methyl-3-cephem-4-carboxylic acid and 0.9 ml of a 33% of a solution of HBr in acetic acid (33%) 6 ml of acetone was added. This mixture was stirred vigorously for 25 minutes while crystals were formed. Filtration, washing with acetone and drying yielded 1.18 g of the title product.

IR (KBr-disc, values in cm⁻¹): 3120, 2820, 2740, 1785, 1725, 1660, 1630, 1525, 1395, 1365, 1285, 1205, 1185, 1115, 1080, 1050, 965, 955, 910, 865, 805, 780, 715, 690, 565, 505.

NMR (360 MHz; CF₃COOD; tetramethylsilane as a reference; δ-values in ppm): 2.18 (s, 3H); 2.52 (s, 3H); 2.64 (s, 3H); 3.21, 3.37 (AB-q, 2H; J= 18 Hz); 5.28, (d, 1H; J = 4.3 Hz); 5.75 (d, 1H; J = 4.3 Hz).

### Example XIII

### Preparation of 4-carboxy-7-isopropylideneammonio-3-methyl-3-cephem bromide from 7-amino-3-methyl-3-cephem-4-carboxylic acid

To a suspension of 1.0 g (purity 98.5%; 4.60 mmoles) of 7-amino-3-methyl-3-cephem-4-carboxylic acid in a mixture of 10 ml of acetic acid and 15 ml of acetone, 0.86 ml of hydrogen bromide solution in water (47%) was added. After filtering off the white crystals formed, washing and drying, 1.47 g of the title product was obtained with a purity of 96%. Yield 92%.

### Example XIV

### Preparation of 4-carboxy-7-cyclohexylideneammonio-3-methyl-3-cephem bromide from 7-amino-3-methyl-3-cephem-4-carboxylic acid

To a solution of 500 mg (2.33 mmoles) of 7-amino-3-methyl-3-cephem-4-carboxylic acid in 0.45 ml of a concentrated HBr solution (47%), 0.50 ml of cyclohexanone was added at ambient temperature. After 20 minutes crystallization started and then during the next 6 hours 2.0 ml of cyclohexanone were added in portions. After standing overnight, the crystals formed were filtered off, washed and dried, yielding 590 mg (67%) of the title product.

IR (KBr-disc, values in cm⁻¹): 3430, 2870, 1796, 1709, 1658, 1630, 1517, 1401, 1354, 1214, 1195, 828, 705.

NMR (360 MHz; CF₃COOD; tetramethylsilane as a reference; δ-values in ppm): 1.63 (m, 2H); 1.92 (m, 4H); 2.17 (s, 3H); 2.78 (m, 4H); 3.25 (d, 1H; J = 16.2 Hz); 3.38 (d, 1H; 16.2 Hz); 5.28 (d, 1H; J = 4.5 Hz); 5.78 (d, 1H; J = 4.5 Hz).

### Example XV

### Preparation of 4-carboxy-7-cyclohexylideneammonio-3-methyl-3-cephem perchlorate from 7-amino-3-methyl-3-cephem-4-carboxylic acid

In an ultrasonic bath 130 mg (0.61 mmole) of 7-amino-3-methyl-3-cephem-4-carboxylic acid was dissolved in 0.175 ml of a perchloric acid solution at 0°C and 0.1 ml of cyclohexanone was added over a period of 30 minutes. After another 30 minutes the crystals were filtered off, washed and dried, giving 171 mg (71%) of the title product.

IR (KBr-disc, values in cm⁻¹): 3430, 3205, 3110, 3010, 1790, 1716, 1658, 1635, 1521, 1410, 1357, 1218, 1196, 1140, 1100, 1050, 835, 703, 621

NMR (360 MHz; CF₃COOD; tetramethylsilane as a reference; δ-values in ppm): 1.58 (m, 2H); 1.87 (m, 4H); 2.13 (s, 3H); 2.73 (m, 4H); 3.21 (s, 2H); 5.20 (d, 1H; J = 4.5 Hz); 5.67 (d, 1H; J = 4.5 Hz).

### Example XVI

### Preparation of 4-carboxy-7-isopropylideneammonio-3-[[(1-methyl-1H-tetrazol-5-yl)thio]methyl]-3-cephem chloride from 7-amino-3-[[(1-methyl-1H-tetrazol-5-yl)thio]methyl]-3-cephem-4-carboxylic acid

To 2 ml of acetic acid saturated with hydrochloric acid 1 g (purity 95%; 2.9 mmoles) of 7-amino-3-[[(1-methyl-1H-tetrazol-5-yl)thio]methyl]-3-cephem-4-carboxylic acid and 3 ml of acetone were added at about 5°C. After stirring at 0°C for 40 minutes the crystalline material was filtered off, washed with acetone and dried giving 1.1 g of the title compound.

IR (KBr-disc, values in cm⁻¹): 2950, 2910, 2830, 1800, 1715, 1655, 1625, 1525, 1470, 1405, 1350, 1250, 1235, 1180, 1110, 1060, 835, 710, 695.

NMR (360 MHz; CF₃COOD; tetramethylsilane as a reference; δ-values in ppm): 2.52 (s, 3H); 2.62 (s, 3H); 3.63, 3.74 (AB-q, 2H; J= 18 Hz); 3.97 (s, 3H); 4.01, 4.51 (AB-q, 2H; J = 13.2 Hz); 5.32 (d, 1H; J = 4.3 Hz); 5.83 (d, 1H; J = 4.3 Hz).

### Example XVII

### Preparation of 4-carboxy-7-isopropylideneammonio-3-[[(1-methyl-1H-tetrazol-5-yl)thio]methyl]-3-cephem bromide from 7-amino-3-[[(1-methyl-1H-tetrazol-5-yl)thio]methyl]-3-cephem-4-carboxylic acid

1 g (purity 95%; 2.9 mmoles) of 7-amino-3-[[(1-methyl-1H-tetrazol-5-yl)thio]methyl]-3-cephem-4-carboxylic acid was added to 0.8 ml of a hydrogen bromide solution (33%) in acetic acid at 0°C. Gradually 10 ml of acetone were added and stirring was continued for 30 minutes at 0°C. After filtration, washing the crystals and drying 1.14 g of the final product was obtained.

IR (KBr-disc, values in cm⁻¹): 3180, 2945, 2900, 2730, 1795, 1720, 1655, 1625, 1515, 1455, 1395, 1370, 1345, 1230, 1175, 1105, 1055, 995, 930, 820, 770, 705.

NMR (360 MHz; CF₃COOD; tetramethylsilane as a reference; δ-values in ppm): 2.52 (s, 3H); 2.64 (s, 3H); 3.64, 3.76 (AB-q, 2H; J= 18 Hz); 3.97 (s, 3H); 4.06, 4.57 (AB-q, 2H; J = 13.2 Hz); 5.37 (d, 1H; J = 4.3 Hz); 5.85 (d, 1H; J = 4.3 Hz).

### Example XVIII

### Preparation of 4-carboxy-7-isopropylideneammonio-3-[[(1-methyl-1H-tetrazol-5-yl)thio]methyl]-3-cephem bromide from 7-amino-3-[[(1-methyl-1H-tetrazol-5-yl)thio]methyl]-3-cephem-4-carboxylic acid

To a suspension of 1.0 g (2.9 mmoles) of 7-amino-3-[[(1-methyl-1H-tetrazol-5-yl)thio]methyl]-3-cephem-4-carboxylic acid in 7 ml of acetone, 0.81 ml of a HBr solution in acetic acid (33%) was added. The reaction mixture was stirred for 30 minutes at 0°C and stored overnight in the refrigerator. Filtration, washing with acetone and drying off the precipitate gave 1.35 g of the title product.

### Example XIX

### Preparation of 4-carboxy-7-isopropylideneammonio-3-vinyl-3-cephem chloride from 7-amino-3-vinyl-3-cephem-4-carboxylic acid

To 0.5 g (2.1 mmoles) of 7-amino-3-vinyl-3-cephem-4-carboxylic acid 2 ml of acetic acid with 7% of hydrochloric acid were added. With stirring a yellow solution was obtained, which solidified gradually. After adding 5 ml of acetone the precipitate dissolved and then a crystalline product was formed. Stirring was continued for 30 minutes at 0°C and the crystals were filtered off, washed with acetone and dried, giving 0.62 g (98%) of the title product.

IR (KBr-disc, values in cm⁻¹): 2980, 2820, 2500, 2000, 1780, 1710, 1660, 1420, 1400, 1370, 1350, 1200, 1175, 1150, 1130, 1060, 995, 935, 845, 720, 695.

NMR (360 MHz; CF₃COOD; tetramethylsilane as a reference; δ-values in ppm): 2.50 (s, 3H); 2.60 (s, 3H); 3.46, 3.52 (AB-q, 2H; J = 18 Hz); 5.28 (d, 1H; J = 4.3 Hz); 5.48 (d, 1H; J = 10.8 Hz); 5.62 (d, 1H; J = 16.8 Hz); 5.74 (d, 1H; J = 4.3 Hz); 7.27 (dd, 1H; J = 10.8 and 16.8 Hz).

### Example XX

### Preparation of 4-carboxy-7-isopropylideneammonio-3-vinyl-3-cephem bromide from 7-amino-3-vinyl-3-cephem-4-carboxylic acid

To 0.4 ml of a hydrogen bromide solution (33%) in acetic acid 0.5 g of 7-amino-3-vinyl-3-cephem-4-carboxylic acid was added at 0°C. After pulverizing the solid material gradually 5 ml of acetone was added with vigorous stirring. Then the reaction mixture was stirred for another 30 minutes at 0°C, and the crystals formed were filtered off, washed with acetone and dried giving 0.62 g (88%) of the title compound.

IR (KBr-disc, values in cm⁻¹): 2940, 2810, 2740, 1795, 1715, 1655, 1610, 1580, 1515, 1395, 1340, 1190, 1170, 1120, 1055, 1020, 990, 935, 800, 775, 725, 690, 425.

NMR (360 MHz; CF₃COOD; tetramethylsilane as a reference; δ-values in ppm): 2.54 (s, 3H); 2.66 (s, 3H); 3.54 (s, 2H); 5.29 (d, 1H; J = 4.3 Hz); 5.51 (d, 1H; J = 10.8 Hz); 5.63 (d, 1H; J = 16.8 Hz); 5.78 (d, 1H; J = 4.3 Hz); 7.32 (dd, 1H; J = 10.8 and 16.8 Hz).

### Example XXI

### Preparation of 4-carboxy-7-cyclohexylideneammonio-3-vinyl-3-cephem bromide from 7-amino-3-vinyl-3-cephem-4-carboxylic acid

To 0.12 ml of a concentrated hydrogen bromide solution (47%) in water subsequently 140 mg (purity 95%; 0.59 mmoles) of 7-amino-3-vinyl-3-cephem-4-carboxylic acid, 0.15 ml of cyclohexanone (1.4 mmole) and 0.1 ml of acetonitrile was added at 0°C. After storing the reaction mixture in the refrigerator overnight, the precipitate formed was filtered off, washed with cyclohexanone and ether and dried, yielding 159 mg (70%) of the title compound.

IR (KBr-disc, values in cm⁻¹): 3440, 2870, 1798, 1710, 1655, 1575, 1517, 1394, 1350, 1212, 978, 943, 702.

NMR (360 MHz; CF₃COOD; tetramethylsilane as a reference; δ-values in ppm): 1.63 (m, 2H); 1.91 (m, 4H); 2.79 (m, 4H); 3.49 (d, 1H; J = 16.2 Hz); 3.57 (d, 1H; 16.2 Hz); 5.32 (d, 1H; J = 4.5 Hz); 5.49 (d, 1H; J = 10.8 Hz); 5.64 (d, 1H; J = 15.1 Hz); 5.82 (d, 1H; J = 4.5 Hz); 7.30 (dd, 1H; J = 10.8 and 15.1 Hz).

### Example XXII

### Preparation of 4-carboxy-7-isopropylideneammonio-3-methyl-1-oxo-3-cephem bromide from 7-amino-3-methyl-3-cephem-1-oxo-4-carboxylic acid

With stirring 0.4 ml of a hydrogen bromide solution (33%) in acetic acid was added to 1 g (2.1 mmoles) of 7-amino-3-methyl-1-oxo-3-cephem-4-carboxylic acid at 0°C. After adding gradually 5 ml of acetone a crystalline precipitate was obtained, which was filtered off, washed with acetone and dried giving 0.60 g (79%) of the title product.

IR (KBr-disc, values in cm⁻¹): 2960, 2740, 1790, 1715, 1550, 1505, 1400, 1360, 1200, 1190, 1150, 1115, 1040, 1010, 980, 910, 845, 810, 790, 720, 690.

NMR (360 MHz; CF₃COOD; tetramethylsilane as a reference; δ-values in ppm): 2.03 (s, 3H); 2.48 (s, 3H); 2.52 (s, 3H); 3.68, 3.82 (AB-q, 2H; J = 18 Hz); 5.42 (d, 1H; J = 4.5 Hz); 6.03 (d, 1H; J = 4.5 Hz).

### Example XXIII

### Preparation of 3-carboxy-6-isopropylideneammonio-2,2-dimethyl-1,1-dioxo-penam chloride from 6-amino-2,2-dimethyl-1,1-dioxo-penam-3-carboxylic acid

In a centrifuge tube 1.0 ml of a hydrogen chloride solution (7%) in acetic acid was added to 0.5 g (2 mmoles) of 6-amino-2,2-dimethyl-1,1-dioxo-penam-3-carboxylic acid at 0°C with stirring. After adding gradually 3 ml of acetone a crystalline precipitate was obtained, which was filtered off, washed with acetone and dried giving 0.55 g (85%) of the title product.

IR (KBr-disc, values in cm⁻¹): 3000, 2900, 2620, 1815, 1760, 1665, 1525, 1465, 1430, 1400, 1325, 1210, 1190, 1150, 1115, 1085, 965, 855, 745, 645, 555.

NMR (360 MHz; CF₃COOD; tetramethylsilane as a reference; δ-values in ppm): 1.68 (s, 3H); 1.81 (s, 3H); 2.88 (s, 3H); 2.90 (s, 3H); 4.90 (s, 1H); 5.54 (d, 1H; J = 4.2 Hz); 6.40 (d, 1H; J = 4.2 Hz).

### Example XXIV

### Preparation of 4-carboxy-7-isopropylideneammonio-3-methyl-2-cephem bromide from 7-amino-3-methyl-2-cephem-4-carboxylic acid

In a centrifuge tube 0.4 ml of a hydrogen bromide solution (33%) in acetic acid was added to 0.5 g (2.3 mmoles) of 7-amino-3-methyl-2-cephem-4-carboxylic acid at 0°C with stirring. Thereafter 2 ml of acetone was added gradually. After precipitation started another 5 ml of acetone was added. After standing overnight at 2°C the crystals formed were filtered off, washed with acetone and dried, giving 0.560 g (73%) of the title product.

IR (KBr-disc, values in cm⁻¹): 2800, 2740, 1775, 1730, 1665, 1370, 1240, 1180, 1150, 1025, 950, 880, 840, 820, 795, 705, 645, 600.

NMR (360 MHz; CF₃COOD; tetramethylsilane as a reference; δ-values in ppm): 1.69 (d, 3H; J ≈ 1 Hz); 2.40 (s, 3H); 2.49 (s, 3H); 4.75 (s, 1H); 5.43 (d, 1H; J = 4.3 Hz); 5.66 (m, 1H); 5.70 (d, 1H; J = 4.3 Hz).

### Example XXV

### Preparation of 4-carboxy-7-isopropylideneammonio-3-methylenecepham bromide from 7-amino-3-methylenecepham-4-carboxylic acid

To 0.8 ml of a hydrogen bromide solution (33%) in acetic acid 1.0 g (purity 96%; 4.49 mmoles) of 7-amino-3-methylene-cepham-4-carboxylic acid was added at 0°C. With stirring a yellow precipitate was obtained. After adding gradually 10 ml of acetone the syrupy material changed into a crystalline product. Stirring for another 30 minutes at 0°C, filtration of the precipitate, washing with acetone and drying yielded 1.30 g (86%) of the title product.

IR (KBr-disc, values in cm⁻¹): 2840 2750, 1775, 1730, 1655, 1525, 1420, 1370, 1230, 1180, 1140, 1075, 935, 800, 790, 730, 590.

NMR (360 MHz; CF₃COOD; tetramethylsilane as a reference; δ-values in ppm): 2.47 (s, 3H); 2.59 (s, 3H); 3.24, 3.53 (AB-q, 2H; J = 14.4 Hz); 5.20 (s, 1H); 5.23 (s, 1H); 5.26 (d, 1H); 5.61 (d, 1H; J = 4.3 Hz); 5.71 (d, 1H; J = 4.3 Hz).

### Example XXVI

### Preparation of N-cyclohexylidene-β-alaninium bromide from β-alanine

Cyclohexanone was added to a solution of β-alanine in a hydrogen bromide solution (33%) in acetic acid. After standing for an hour in the refrigerator the formed crystals were filtered off, washed with cyclohexanone and ether, and dried.

IR (KBr-disc, values in cm⁻¹): 3440, 2930, 2555, 1740, 1680, 1527, 1458, 1371, 1323, 1210, 980, 947, 878, 859, 828, 797, 668, 629.

NMR (360 MHz; CF₃COOD; tetramethylsilane as a reference; δ-values in ppm): 1.67 (m, 2H); 1.87 (m, 4H); 2.72 (m, 4H); 2.98 (t, 2H); 4.03 (m, 2H).

### Example XXVII

### Preparation of 3-acetoxymethyl-4-carboxy-7-isopropylideneammonio-3-cephem chloride from the sodium salt of cephalosporin C

To a suspension of 12.4 g (purity 95%; 24.8 mmoles) of the sodium salt of cephalosporin C dihydrate in 115 ml methylenechloride and 19.4 ml (153 mmoles) of N,N-dimethylaniline, 18 ml (141.5 mmoles) of trimethylsilyl chloride was added gradually. After stirring the reaction mixture at 35°C during 90 minutes the temperature was lowered to -50°C. 7.5 g (36 mmoles) of phosphorus pentachloride were added and stirring was continued at -40°C for 2 hours. After the temperature was lowered to -50°C, 38 ml of methanol were added and stirring was continued at -30°C for 90 minutes. To the reaction mixture 200 ml of acetone were added during 10 minutes. After stirring for another hour at 2°C the crystalline precipitate formed was filtered off, washed with acetone and dried, yielding 9.86 g of the title product. Purity 70%. Yield 80%.

### Example XXVIII

### Preparation of 3-acetoxymethyl-4-carboxy-7-cyclohexylideneammonio-3-cephem chloride from the sodium salt of cephalosporin C

According to the same method as described in the example XXVII the title product was prepared by adding 90 ml of cyclohexanone instead of 200 ml of acetone, concentrating the reaction mixture and adding 25 ml of a HCl solution in water (37%).

### Example XXIX

### Preparation of 3-acetoxymethyl-4-carboxy-7-cyclohexylideneammonio-3-cephem bromide from cephalosporin C

24,7 g of cephalosporin C (purity 96%; 50 mmoles) were silylated with a mixture of 35.9 ml (283 mmoles) of trimethylsilylchloride and 38.7 ml (305 mmoles) of N,N-dimethylaniline in 225 ml of methylene chloride at 35°C during 90 minutes with stirring. After cooling down to -50°C, 14.7 g (70.5 mmoles) of phosphorus pentachloride were added and stirring was continued at -40°C for 100 minutes. After the temperature was lowered to -50°C, 75 ml of methanol were added and stirring was continued at -30°C for 90 minutes.

To 50 ml of this reaction mixture 25 ml of chilled (0°C) cyclohexanone were added, methanol and methylene chloride were evaporated and 5 ml of a HBr solution in water (47%) were added. After keeping the temperature at 0°C for about 190 minutes and at -20°C for 30 minutes the crystalline precipitate was isolated by centrifugation, washed with cyclohexanone and acetone and dried, giving 2.68 g of the title product. Purity 84%. Yield 77%.

### Example XXX

### Preparation of 3-methyl-4-carboxy-7-isopropylideneammonio-3-cephem chloride from 7-phenylacetamido-3-methyl-3-cephem-4-carboxylic acid

12 g of 7-phenylacetamido-3-methyl-3-cephem-carboxylic acid (34.6 mmoles) were silylated with a mixture of 15.1 ml (119.1 mmoles) of N,N-dimethylaniline and 8.3 ml (65.3 mmoles) of trimethylsilyl chloride in 100 ml of methylene chloride at 10°C during 5 minutes with stirring. After cooling down to -65°C 8.3 g (39.9 mmoles) of phosphorus pentachloride were added and stirring was continued at -30°C for 100 minutes. After the temperature was lowered to -65°C, a mixture of 55 ml of isobutyl alcohol and 200 ml of acetone was added and stirring was continued at -30°C for 30 minutes. Then the temperature raised to 0°C stirring was continued for 4 hours and the precipitate was filtered off, washed and dried, giving 8.9 g (85%) of the title product. A sample (2.5 g) was purified by dissolving this product in cold formic acid, adding some isobutyl alcohol and phosphorus pentachloride, filtering off and drying the precipitate, giving 2.1 g of the pure product.

### Example XXXI

### Preparation of 7-amino-3-[[(1-methyl-1H-tetrazol-5-yl)thio]methyl]-3-cephem-4-carboxylic acid from 3-acetoxymethyl-4-carboxy-7-cyclohexylideneammonio-3-cephem bromide

To a mixture of 0.437 g (1.01 mmoles) of 3-acetoxymethyl4-carboxy-7-cyclohexylideneammonio-3-cephem bromide, 0.283 g (2.43 mmoles) of 2-mercapto-1-methyl-1H-tetrazole and 5 ml of acetonitrile, 0.62 ml of trifluoromethanesulfonic acid in 5 ml of acetonitrile was added at room temperature. After stirring for 40 minutes 10 ml of ice water and 10 ml of acetone were added. Then the pH was adjusted to 3.7 with ammonia (ice bath) and the reaction mixture was kept overnight in the refrigerator. The precipitate formed was filtered off, washed with water and acetone and dried. Isolated was 0.248 g of 7-amino-3-[[(1-m-ethyl-1H-tetrazol-5-yl)thio]methyl]-3-cephem-4-carboxylic acid having a purity of 91%. Yield 88%.

IR-spectrum (KBr-disc; values in cm⁻¹): 3440, 2620, 1804, 1617, 1540, 1512, 1413, 1389, 1350, 1293, 1229, 1172, 1119, 1062, 1009, 803, 790, 700, 690 and 430.

NMR-spectrum (60 MHz; CD₂O₂; sodium 3-(trimethylsilyl)-1-propane sulfonate as a reference; δ-values in ppm): 3.40 and 3.84 (AB-q, 2H; J = 12 Hz); 4.00 (s, 3H); 3.98 and 4.35 (AB-q, 2H; J = 9 Hz); 5.02 (d, 1H; J = 4.5 Hz); 5.40 (d, 1H; J = 4.5 Hz).

### Example XXXII

### Preparation of 7-ammonio-4-carboxy-3-[(1-pyridinio)methyl]-3-cephem diiodine from 3-acetoxymethyl-4-carboxy-7-isopropylideneammonio-3-cephem chloride

To a suspension of 1.4 g (purity 90%; 3.62 mmoles) of 3-acetoxymethyl-4-carboxy-7-isopropylideneammonio-3-cephem chloride in 20 ml of methylene chloride 3.0 ml of N,O-bis(trimethylsilyl)trifluoroacetamide were added at room temperature. The silylation was accomplished by refluxing for 120 minutes. After the solution was cooled in an ice bath, 1.14 ml (8 mmoles) of trimethylsilyl iodide was added, the temperature was raised to 22°C and stirring was continued for 30 minutes. The reaction mixture was cooled again (2°C), 1.93 ml (24 mmoles) of pyridine was added and the solution was stirred at 2°C for 20 minutes. After addition of 1.6 ml of methanol and 0.2 ml of water during 20 minutes and stirring for another 10 minutes, the crystals formed were filtered off, washed with methylene chloride and dried, giving 2.11 g of the title product with a purity of 75%. Yield 80%.

IR-spectrum (KBr-disc; values in cm⁻¹): 3400, 1785, 1735, 1530, 1480, 1400, 1350, 1155, 745 and 675.

NMR-spectrum (60 MHz; CD₂O₂; sodium 3-(trimethylsilyl)-1-propanesulfonate as a reference; δ-values in ppm): 3.21, 3.52, 3.66 and 3.96 (AB-q, 2H; J = 18.5 Hz); 5.20 and 5.29 (d, J = 5.2 Hz); 5.34 and 5.42 (d, 1H; J = 5.2 Hz); 5.29, 5.54, 5.75 and 5.95 (AB-q, 2H; J = 14.7 Hz); 7.97 to 9.14 (m, 5H).

## Claims

1. A compound which is an amino carboxylic acid derivative comprising, as an N-terminal group, a (cyclo)alkylideneammonio group of formula (II): 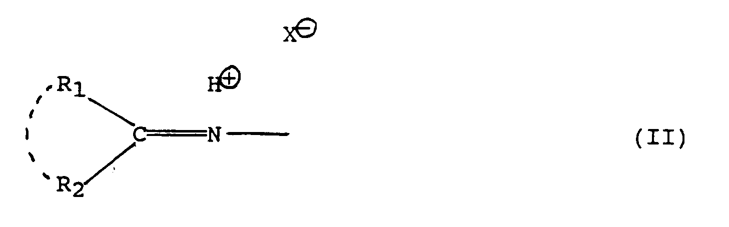 wherein
X^{⊖} is an anion from an acid HX,
R₁ and R₂ are the same or different and each is a C₁-C₁₆ alkyl group, or R₁ and R₂ together with the carbon atom to which they are attached form a cycloalkylidene ring with up to 8 carbon atoms, with the proviso that the compound is not 7β-(cyclo)alkylideneammonio-3-halomethyl-3-cephem-4-carboxylic acid.

2. A compound according to claim 1 which is a β-lactam derivative.

3. A compound according to claim 2 having the following formula (I): 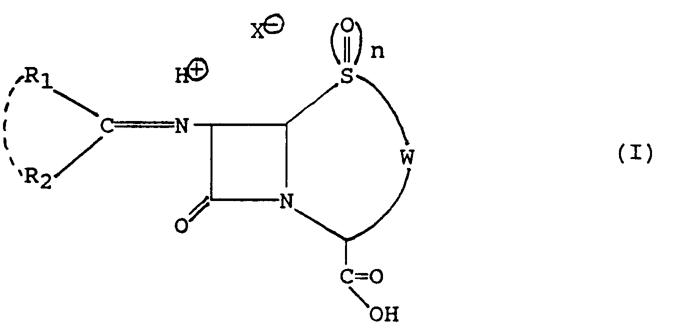 wherein
W is 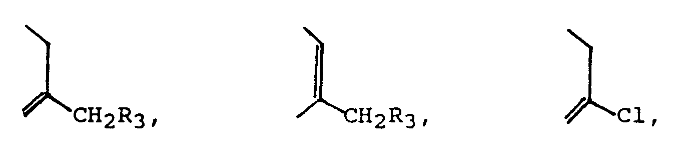 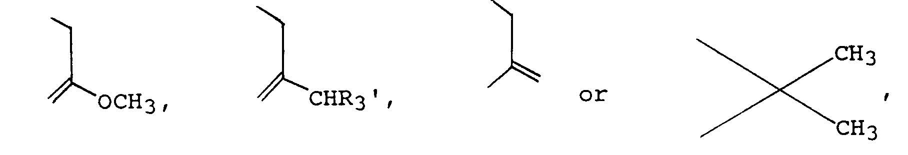 R₃ is hydrogen, (lower)alkoxy, (lower)alkylthio, (lower)alkanoyloxy, (lower)alkanoylthio or S-R₄, where R₄ is pyridine, pyrimidine, pyridazine, pyrrole, imidazole, pyrazole, isoxazole, isothiazole, thiazole, triazole, oxadiazole, thiadiazole, triazine, thiatriazole or tetrazole linked by a ring carbon atom to the sulphur atom, unsubstituted or substituted on a ring carbon atom by one or more of the following groups, viz. cyano, chloro, di(lower)alkylamino, (lower)alkyloxy, (lower)alkyloxycarbonyl, di(lower)alkylcarbamoyl, hydroxy, sulfo, carboxy or optionally substituted (lower)alkyl or substituted on one or more ring nitrogen atoms by an optionally substituted (lower)alkyl group whereby the substituents attached to the first or the second carbon atom of a (lower)alkyl group attached either to a ring carbon or ring nitrogen atom are di(lower)alkylamino, chloro, cyano, methoxy, (lower)alkyloxycarbonyl, N,N-dimethylcarbamoyl, hydroxy, carboxy or sulfo,
R₃' is (lower)alkylidene,
n is 0, 1 or 2 and
R₁, R₂ and X are as defined in claim 1, and the term "lower" refers to an alkyl group with up to 8 carbon atoms.

4. A compound according to claim 3,
wherein
W is 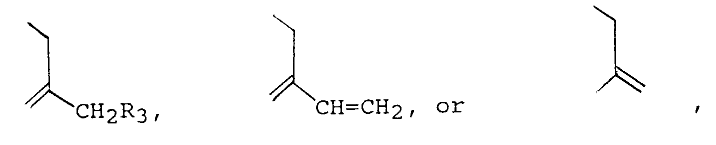 where R₃ is hydrogen, acetoxy, (1-methyl-1H-tetrazol-5-yl)thio and n is 0 or 1 or
W is 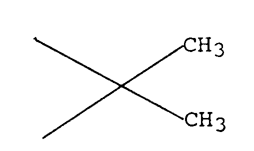 and n is 2,
and wherein
X is Cl⁻, Br⁻, I⁻, ClO₄⁻, HSO₄⁻, or CH₃COO⁻, and
R₁ is methyl, R₂ is methyl or
R₁ and R₂ together with the carbon atom to which they are attached form cyclopentylidene or cyclohexylidene.

5. A process for the preparation of an amino carboxylic acid derivative, characterized in that the N-terminal amine group of an amino carboxylic acid is converted into a group of formula (II) as defined in claim 1 by adding a ketone R₁R₂CO to the amino carboxylic acid in the presence of an acid HX, R₁, R₂ and X being as defined in claim 1.

6. A process according to claim 5, wherein the starting amino carboxylic acid is protected at its N-terminus, which comprises deprotecting the N-terminal amine and adding the ketone R₁R₂CO thereto in situ.

7. A process according to claim 6, characterized in that a compound of formula (I) as defined in claim 3, is prepared from a protected amino carboxylic acid of formula (III), 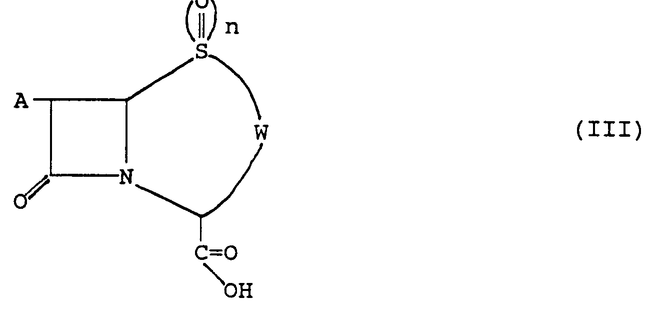 wherein
W and n are as defined in claim 3, and
A is a protected amino group,
by carrying out the following successive steps:
(a) silylating the starting amino carboxylic acid,
(b) converting the product of step (a) into an imidoyl chloride,
(c) reacting the imidoyl chloride with an alcohol to form the corresponding iminoether and/or the 7-amino derivative, and
(d) adding a ketone R₁R₂CO.

8. A process according to claim 7 wherein each of steps (a) to (d) is performed in the same reaction vessel without isolation of the intermediate products.

9. A process for the preparation of an antibiotic which comprises preparing, as an intermediate, a compound as claimed in any one of claims 1 to 4.

10. A process according to claim 9 which comprises preparing the intermediate by a process as claimed in any one of claims 5 to 8.

## Patentansprüche

1. Verbindung, bestehend aus einem Aminocarbonsäure-derivat, welches als N-terminale Gruppe eine Cycloalkyliden-ammoniogruppe der Formel (II): 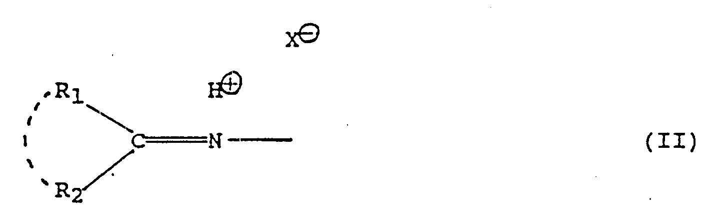 in welcher X^{⊖} das Anion einer Säure HX bedeutet, R₁ und R₂ identisch oder unterschiedlich sind und jeweils eine C₁-C₁₆-Alkylgruppe bedeuten oder R₁ und R₂ zusammen mit dem Kohlenstoffatom, an das sie gebunden sind, einen Cycloalkylidenring mit bis zu 8 Kohlenstoffatomen bilden, mit der Massgabe, dass die Verbindung keine 7β-Cycloalkylidenammonio-3-halogenomethyl-3-cephem-4-carbonsäure ist.

2. Verbindung nach Anspruch 1, welche ein β-Lactamderivat ist.

3. Verbindung nach Anspruch 2, welche die folgende Formel (I) hat: 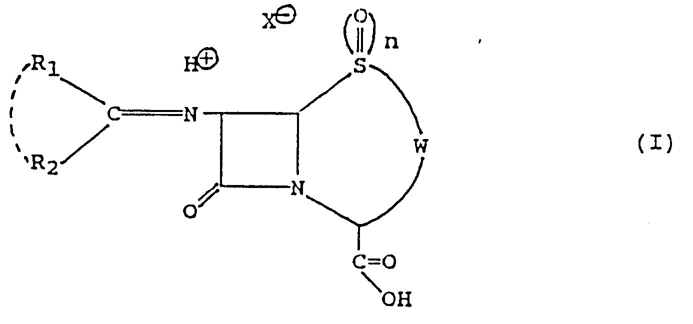 in welcher W 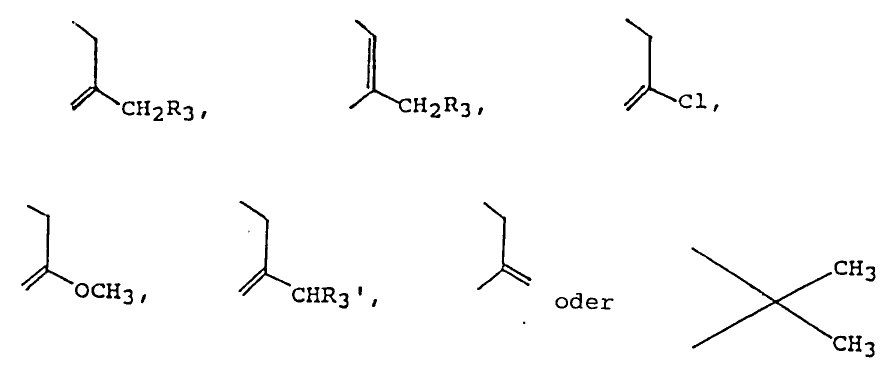 darstellt, R₃ Wasserstoff, niederes Alkoxy, niederes Alkylthio, niederes Alkanoyloxy, niederes Alkanoylthio oder S-R₄ bedeutet, worin R₄ Pyridin, Pyrimidin, Pyridazin, Pyrrol, Imidazol, Pyrazol, Isoxazol, Isothiazol, Thiazol, Triazol, Oxadiazol, Thiadiazol, Triazin, Thiatriazol oder Tetrazol bedeutet, welche an das Schwefelatom durch ein Kohlenstoffatom des Rings gebunden sind, wobei ein Kohlenstoffatom des Rings unsubstituiert oder durch eine oder mehrere der folgenden Gruppen substituiert ist, nämlich Cyano, Chloro, Di(niederalkyl)amino, niederes Alkoxy, niederes Alkoxycarbonyl, Di(niederalkyl)carbamoyl, Hydroxy, Sulfo, Carboxy oder gegebenenfalls substituiertes niederes Alkyl, oder wobei eines oder mehrere Stickstoffatome des Ringes durch eine gegebenenfalls substituierte niedere Alkylgruppe substituiert ist/sind, wobei die Substituenten des ersten oder des zweiten Kohlenstoffatoms einer an einem Kohlenstoffatom oder einein Stickstoffatom des Ringes haftenden niederen Alkylgruppe Di(niederalkyl)amino, Chloro, Cyano, Methoxy, niederes Alkoxycarbonyl, N,N-Dimethylcarbamoyl, Hydroxy, Carboxy oder Sulfo sind, R₃' niederes Alkyliden bedeutet, n die Zahl 0, 1 oder 2 bedeutet und R₁, R₂ und X wie in Anspruch 1 definiert sind und das Wort "niederes" eine Alkylgruppe mit bis zu 8 Kohlenstoffatomen bedeutet.

4. Verbindung nach Anspruch 3, worin W 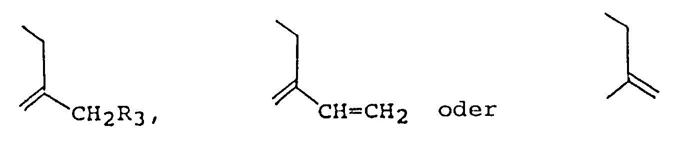 darstellt, worin R₃ Wasserstoff, Acetoxy, (1-Methyl-1H-tetrazol-5-yl)thio bedeutet und n die Zahl 0 oder 1 ist oder W 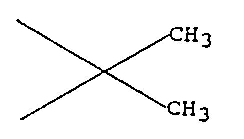 darstellt und n die Zahl 2 ist und worin X Cl⁻, Br⁻, I⁻, ClO₄⁻, HSO₄⁻ oder CH₃COO⁻ bedeutet und R₁ Methyl, R₂ Methyl bedeutet oder R₁ und R₂ zusammen mit dem Kohlenstoffatom, an welches sie gebunden sind, eine Cyclopentyliden- oder Cyclohexylidengruppe bilden.

5. Verfahren zur Herstellung eines Aminocarbonsäurederivates, dadurch gekennzeichnet, dass die N-terminale Aminogruppe einer Aminocarbonsäure in eine Gruppe der wie in Anspruch 1 definierten Formel (II) dadurch umgewandelt wird, dass ein Keton R₁R₂CO der Aminocarbonsäure in Gegenwart einer Säure HX, wobei R₁, R₂ und X wie in Anspruch 1 definiert sind, zugegeben wird.

6. Verfahren nach Anspruch 5, in welchem die als Ausgangsprodukt verwendete Aminocarbonsäure an ihrem N-Ende geschützt ist, dadurch gekennzeichnet, dass man die Schutzgruppe des N-terminalen Amins abspaltet und das Keton R₁R₂CO in situ zusetzt.

7. Verfahren nach Anspruch 6, dadurch gekennzeichnet, dass eine Verbindung der wie in Anspruch 3 definierten Formel (I) aus einer geschützten Aminocarbonsäure der Formel (III) hergestellt wird, 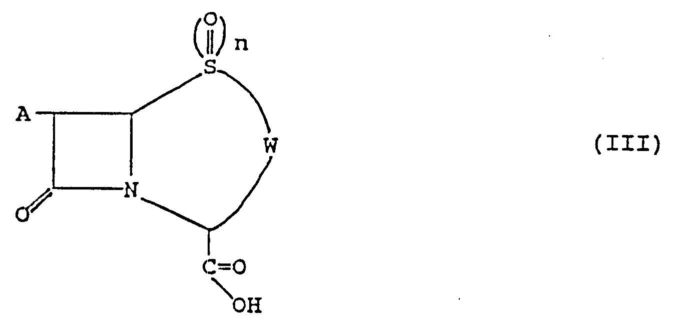 in welcher W und n wie in Anspruch 3 definiert sind und A eine geschützte Aminogruppe darstellt, indem man hintereinander die folgenden Stufen ausführt:
(a) die Silylierung der als Ausgangsprodukt verwendeten Aminocarbonsäure,
(b) die Umwandlung des Produktes der Stufe (a) in ein Imidoylchlorid,
(c) die Reaktion des Imidoylchlorids mit einem Alkohol zur Bildung des entsprechenden Iminoethers und/oder des entsprechenden 7-Aminoderivates und
(d) der Zusatz eines Ketons R₁R₂CO.

8. Verfahren nach Anspruch 7, in welchem jede der Stufen (a) bis (d) in demselben Reaktionsgefäss ohne Isolierung der Zwischenprodukte durchgeführt wird.

9. Verfahren zur Herstellung eines Antibiotikums, dadurch gekennzeichnet, dass man als Zwischenprodukt eine Verbindung nach irgendeinem der Ansprüche 1 bis 4 herstellt.

10. Verfahren nach Anspruch 9, dadurch gekennzeichnet, dass man das Zwischenprodukt durch ein Verfahren nach irgendeinem der Ansprüche 5 bis 8 herstellt.

## Revendications

1. Composé qui est un dérivé d'un acide aminocarboxylique comprenant comme radical N-terminal, un radical (cyclo)alcoylidèneimminium de formule (II) : 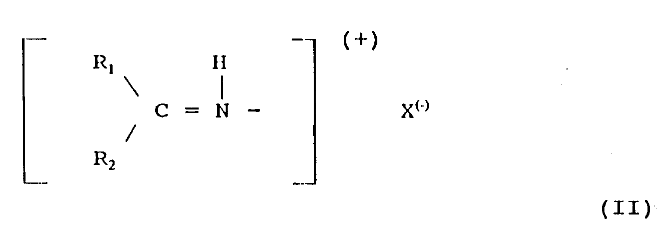 où
X^{⊖} est un anion provenant d'un acide HX;
R₁ et R₂ sont identiques ou différents et chacun est un radical alcoyle en C₁ à C₁₆ ou R₁ et R₂ avec l'atome de carbone auquel ils sont fixés forment ensemble un cycloalcoylidène de jusqu'à 8 atomes de carbone, à la condition que le composé ne soit pas l'acide 7β-(cyclo)alcoylidèneimminio-3-halométhyl-3-céphèm-4-carboxylique.

2. Composé suivant la revendication 1, qui est un dérivé β-lactame.

3. Composé suivant la revendication 2, ayant la formule (I) suivante : 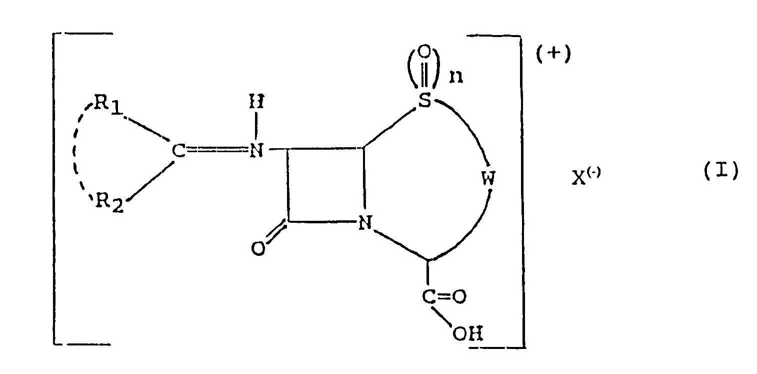 où
W est : 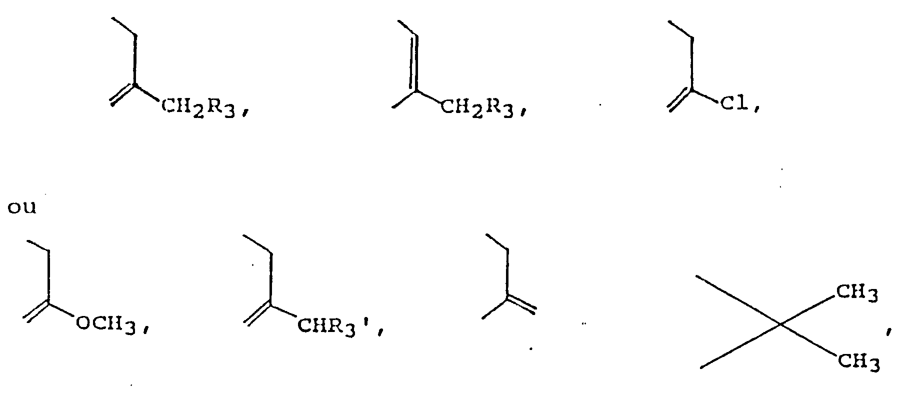 R₃ est hydrogène, alcoxy inférieur, alcoylthio inférieur, alcanoyloxy inférieur, alcanoylthio inférieur ou S-R₄, dans lequel R₄ est pyridine, pyrimidine, pyridazine, pyrrole, imidazole, pyrazole, isoxazole, isothiazole, thiazole, triazole, oxadiazole, thiadiazole, triazine, thiatriazole ou tétrazole fixé par un atome de carbone du cycle à l'atome de soufre, non substitué ou substitué sur un atome de carbone cyclique par un ou plusieurs des radicaux suivants, à savoir cyano, chloro, di(alcoyl inférieur)amino, alcoyloxy inférieur, (alcoyl inférieur)oxycarbonyle, di(alcoyl inférieur)carbamoyle, hydroxy, sulfo, carboxy ou alcoyle inférieur facultativement substitué, ou substitué sur un ou plusieurs atomes d'azote du cycle par un radical alcoyle inférieur facultativement substitué où les substituants fixés au premier ou au deuxième atome de carbone d'un radical alcoyle inférieur fixé à un atome de carbone ou d'azote cyclique, sont di(alcoyl inférieur)amino, chloro, cyano, méthoxy, (alcoyl inférieur)oxycarbonyle, N,N-diméthylcarbamoyle, hydroxyle, carboxy ou sulfo;
R₃' est alcoylidène inférieur;
n est 0, 1 ou 2, et
R₁, R₂ et X sont comme défini à la revendication 1, et le terme "inférieur" se réfère à un radical alcoyle de jusqu'à 8 atomes de carbone.

4. Composé suivant la revendication 3, dans lequel :
W est : 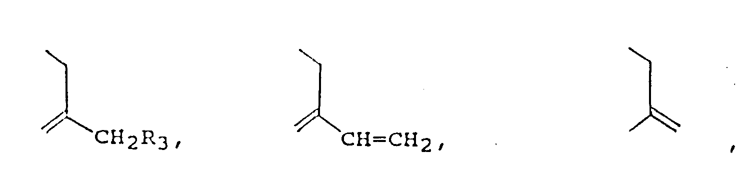 où R₃ est hydrogène, acétoxy, (1-méthyl-1H-tétrazol-5-yl)thio et n est 0 ou 1, ou
W est : 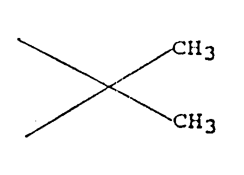 et n est 2,
et dans lequel
X^{⊖} est Cl⁻, Br⁻, I⁻, ClO₄⁻, HSO₄⁻ ou CH₃COO⁻, et
R₁ est méthyle et R₂ est méthyle, ou
R₁ et R₂ avec l'atome de carbone auquel ils sont fixés forment ensemble un cyclopentylidène ou un cycloheyylidène.

5. Procédé de préparation d'un dérivé d'un acide aminocarboxylique, caractérisé en ce que le radical amino N-terminal d'un acide aminocarboxylique est converti en un radical de formule (II) défini à la revendication 1, par addition d'une cétone R₁R₂CO à l'acide aminocarboxylique en présence d'un acide HX, R₁, R₂ et X étant définis à la revendication 1.

6. Procédé suivant la revendication 5, dans lequel l'acide aminocarboxylique de départ est protégé sur son extrémité N, qui comprend la déprotection de l'amine N-terminale et l'addition de la cétone R₁R₂CO in situ.

7. Procédé suivant la revendication 6, caractérisé en ce qu'un composé de formule (I) défini à la revendication 3 est préparé à partir d'un acide aminocarboxylique protégé de formule (III) : 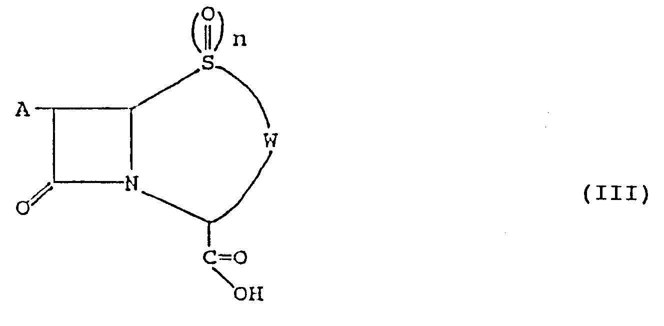 dans lequel :
W et n sont comme défini à la revendication 3, et
A est un radical amino protégé,
en réalisant les étapes successives suivantes :
(a) silylation de l'acide aminocarboxylique de départ;
(b) conversion du produit de l'étape (a) en un chlorure d'imidoyle;
(c) réaction du chlorure d'imidoyle avec un alcool pour former l'imminoéther correspondant et/ou le dérivé 7-amino, et
(d) addition d'une cétone R₁R₂CO.

8. Procédé suivant la revendication 7, dans lequel chacune des étapes (a) à (d) est réalisée dans le même récipient de réaction sans isolement des produits intermédiaires.

9. Procédé de préparation d'un antibiotique qui comprend la préparation d'un composé suivant l'une quelconque des revendications 1 à 4, en tant qu'intermédiaire.

10. Procédé suivant la revendication 9, qui comprend la préparation de l'intermédiaire par un procédé suivant l'une quelconque des revendications 5 à 8.
